# EUROPEAN PATENT APPLICATION

(11) **EP 4 046 646 A1**
(43) Date of publication of application: **24.08.2022**
(21) Application number: 22158128.3
(22) Date of filing: 23.02.2022
(51) Int. Cl.: A61K 36/074, A61K 31/715, A61K 31/716, A61K 36/28, A61K 9/02, A61K 45/06, A61P 15/02, A61P 31/20

(54) **PHARMACEUTICAL COMPOSITION FOR THE TREATMENT OF HPV INFECTIONS**

(30) Priority: 23.02.2021 IT 202100004214
(71) Applicant: Zetemia S.r.l., 80038 Pomigliano d'Arco (NA) (IT)
(72) Inventor: PICCOLO, Federico, 80038 Pomigliano d'Arco NA (IT); DESIDERIO, Vincenzo, 80137 Napoli (IT); DE FRANCISCIS, Pasquale, 81100 Caserta (IT); PRISCO, Claudia, 80056 Ercolano NA (IT); GRIECO, Paolo, 80121 Napoli (IT); STRADELLA, Cristina, 80129 Napoli (IT); PEPE, Giuseppe, 80011 ACERRA NA (IT)
(74) Representative: Cattaneo, Elisabetta

(57) **Abstract**

The present invention concerns a composition comprising: - from1 to 5% by weight of one or more beta-glucans, - from 1 to 5% by weight of a prebiotic compound, - from 1 to 8% by weight of a dry extract of Ganoderma Lucidum Reishi, wherein said dry Ganoderma Lucidum Reishi has a polysaccharide titer of 50%, and - from 2 to 8% by weight of a fat-soluble extract of echinacea, being the amounts expressed with respect to the total weight of the composition, and wherein said composition is for use in the treatment of HPV (Papilloma virus) vaginal infections and symptoms associated thereto by topical vaginal administration.

## Description

### FIELD OF THE INVENTION

The present invention relates to a topical composition to be used through vaginal administration in the prevention and/or treatment of vaginal or cervical disorders caused by human papilloma virus (infectious agent) comprising a dry extract of Ganoderma Lucidum Reishi, and therefore in the prevention of disorders related thereto. The composition of the invention acts by strengthening the genital immunity against such infectious pathogens and promoting a consistent re-epithelialization of the damaged tissue, thus restoring a healthy vaginal microenvironment.

### STATE OF THE ART

Gynecological infections are widespread among women and involve various age groups. The most common disorders include bacterial vulvovaginitis, vaginal yeast infection, trichomoniasis, nonspecific vulvovaginitis, and viral infection. It is now known that viral infections are among the most difficult to treat, and in particular human papilloma virus (HPV) infection, in some cases responsible for cervical cancer, is a growing source of concern especially among sexually active women. According to widely established statistics, around 500,000 women are diagnosed with invasive cervical cancer each year worldwide and 250,000 women die from it, mainly in developing countries. About 20% of women who experience HPV infection in the cervix for one year will develop cervical intraepithelial neoplasia or cervical cancer over the following five years. HPV genotypes 16 and 18 are responsible for approximately 70% of cervical cancers, with the remaining 30% caused by other equally cancerous HPV types. HPV16 represents the "most cancerous" in terms of number of cases of cervical intraepithelial neoplasms or cervical cancer. The low-risk genotypes 6 and 11, on the other hand, cause most of the genital warts manifestations. Although HPV infections represent the most common sexually transmitted viral infection, most of them are transient and asymptomatic, with approximately 70% spontaneous regression (Oakeshott, P. et al. Frequency and risk factors for prevalent, incident, and persistent genital carcinogenic human papillomavirus infection in sexually active women: Community based cohort study. BMJ 345, (2012)).

Human papilloma viruses (HPVs) consist of a heterogeneous group of viruses (30-40 different types of HPV identified to date) known to induce a variety of squamous cell tumors (papillomas and warts) in the skin and on the mucous membranes of the respiratory, gastrointestinal, and genitourinary tracts. In the genital tract, the venereal wart (Condyloma acuminatum) has been recognized since ancient times and known to be a sexually transmitted disease (STD). In 1976, two more morphologically distinct HPV lesions were described in the uterine cervix. Subsequently, these new HPV lesions have been shown to be frequently associated with concomitant cervical intraepithelial neoplasia (CIN) and carcinoma in situ (CIS), and occasionally also with invasive cervical carcinomas. These morphological findings, supported by the growing number of reports of malignant transformation of HPV lesions, as well as data from animal experiments and epidemiological investigations, have supported the concept that HPV is involved in the development of cervical carcinomas. Viral DNA integration into the host genome has been postulated as an important etiological event during cervical carcinogenesis. High-risk HPV DNA integration often results in the deletion or disruption of the large fragment of the E1 and E2 regions and the overexpression of oncogenes E6 and E7 in the viral genome, and in the activation of oncogenes and inactivation of tumor suppressors in the host genome. Recent studies have shown that hrHPV (high-risk human papilloma virus) supplementation can be used as a predictive biomarker in cervical lesion screening. The most effective diagnostic approaches are based on fluorescence *in situ* hybridization, quantitative real-time PCR, and Sanger sequencing of hybrid viral DNA (Huang, S.-S., Hao, D.-Z., Zhang, Y., Liu, H.-M. & Shan, W.-S. Progress in studies of the mechanisms and clinical diagnosis of cervical carcinoma associated with genomic integration of high-risk human papillomavirus DNA. Prog. Stud. Mech. Clin. diagnosis Cerv. carcinoma Assoc. with genomic Integr. high-risk Hum. papillomavirus DNA. 39, 775-783 (2017)). The incidence and mortality of cervical cancer, however, have declined in developed countries thanks to the discovery of the Pap smear in the 1940s, which allowed for the rapid identification of morphological changes in the cervical epithelium. The use of the Pap smear in national screening programs can be dated to the 1960s and 1970s and is still a milestone in most of current programs. In addition, the International Agency for Research on Cancer (IARC) has determined that the incidence of invasive cervical cancer can be reduced by at least 80% with the implementation of cervical cancer screening programs based on Pap smears every three to five years for women between the ages of 35 and 64.

The identification of HPV as the main etiological agent of cervical cancer has presented new opportunities for the development of preventive modalities (Mushegian, A. R. Are there 1031 virus particles on earth, or more, or fewer? J. Bacteriol. 202, (2020)).

Two decades of efforts culminated in 2006 with the approval of the first safe and effective HPV prophylactic vaccine. The first approved vaccine was the quadrivalent Gardasil/Silgard, which targets HPV6, 11,16 and 18. One year later, the bivalent Cervarix vaccine targeting HPV16 and 18 was approved and, more recently, the nonavalent Gardasil 9 vaccine, which targets HPV6, 11, 16, 18, 31, 33, 45, 52 and 586-7-8, was also approved. All three of these vaccines target HPV16 and 18 and contain hpv L1protein virus-like particles (VLPs) expressed in different cell types. VLPs are morphologically and antigenically similar to native HPV virions and, due to the genomic similarity between different types of viruses, a certain degree of protection against HPV types not targeted by the vaccine is also achieved, this is the "so-called cross-protection". HPV vaccines elicit immunity through the production of high titers of anti-HPV IgG neutralizing antibodies, which block virus entry into host cells. Quadrivalent and nonavalent vaccines contain VLPs of two hrHPV types, 6 and 11, which are responsible for over 90% of anogenital warts and laryngeal papillomas. Furthermore, the nonavalent vaccine is targeted against the five types (HPV31, 33, 45, 52, 58) most frequently identified in cervical cancer after HPV16 and 18. However, even with the cross-protection and increased number of HPV types covered by the non-equivalent vaccine, HPV vaccines do not protect against all types of HPV that cause cervical cancer (Higgins, L. M. et al. Adolescents' intention and self-efficacy to follow Pap testing recommendations after receiving the HPV vaccine. Hum. Vaccin. Immunother. 12, 1498-1503 (2016)). In light of what has been stated so far, despite HPV being a problem with a high incidence, there is no real therapy for it, but in most cases the infection undergoes spontaneous resolution over a few years. Medical therapy against the HPV virus with drugs is mostly aimed at strengthening the immune system, with the help of supplements containing all the nutrients necessary for the human body daily needs. Currently, the prescription of the food supplement known as "Factor M", based on fermented Papaya, Beta-glucans, Vitamin C, Vitamin D3, Vitamin E and Zinc, useful in case of deficiency or increased needs of these nutrients, is widespread. Fermented Papaya is known for its antioxidant properties and as support for the body's natural defenses. Beta-glucans are natural substances of plant origin which, once taken, are not metabolized by our enzymes and, therefore, cannot be absorbed in the intestine. During the passage through the intestinal lumen, beta-glucans are recognized as a non-self molecules by the immune system, which becomes alerted and prepares to fight any viruses/bacteria. The presence of Zinc and Vitamins C, D3 and E protect cells from oxidative stress. In addition, Vitamin C, Vitamin D and Zinc contribute to the normal function of the immune system.

Recently a new class of active ingredients has been added, the so-called "Immune Response Modifiers", modulators of the immune response, among which we find imiquimod, marketed under the name Aldara^{®}, able to stimulate the production of interferon with antiviral function. Although these drugs show a certain degree of efficacy, they are not free from adverse reactions related to the development of inflammation locally, in the area of application, an effect that can compromise therapy continuation.

Another therapeutic strategy, reserved for lesions from visceral HPV viruses (e.g. respiratory tract, bladder, rectum, etc.) as well as for the strains most at risk, 16 - 18, or for immunosuppressed subjects, involves the administration of interferon systemically (subcutaneous use). Despite being a natural substance, it is in fact produced by leukocytes, this drug generates flu-like side effects such as fever, fatigue, and muscle-joint pain. Therefore, up to now its systemic use against HPV, although very effective, has remained limited. Many therapeutic plans, instead, provide for the administration of Isoprenol (also available in vaginal ovules) which, despite having only a weak action against the HPV virus, has no side effects and not a high cost. In addition to the use of drugs, there is a medical anti-HPV virus therapy with homeopathic drugs, including Transfactor 11, which is proposed as a drug for the prophylactic approach of relapses, to be associated with surgical therapy.

The object of the present invention is therefore to provide a treatment of vaginal infections that is both effective and achieves patient's compliance.

### SUMMARY OF THE INVENTION

The above object was achieved by a composition comprising:
- from1 to 5% by weight of one or more beta-glucans,
- from 1 to 5% by weight of a prebiotic compound,
- from 1 to 8% by weight of a dry extract of Ganoderma Lucidum Reishi, wherein said dry extract of Ganoderma Lucidum Reishi has a polysaccharide titer of 50%, and
- from 2 to 8% by weight of a fat-soluble extract of echinacea,
being the amounts expressed with respect to the total weight of the composition, and wherein said composition is for use in the treatment of HPV (Papilloma virus) vaginal infections and symptoms associated thereto by topical vaginal administration.

The present invention therefore relates to a specific composition comprising a dry extract of Ganoderma Lucidum, named Reishi, for use in the treatment of vaginal HPV (Papilloma virus) infections and symptoms associated thereto by topical vaginal administration.

Without being bound to any theory, the inventors of the present invention have found the synergistic combination of active ingredients that allows the treatment of HPV (Papilloma virus) vaginal infections and symptoms associated thereto by topical vaginal administration.

The composition of the invention comprises a dry extract of Ganoderma Lucidum Reishi having a polysaccharide titer of 50% and a fat-soluble extract of echinacea in definite amounts. These extracts together with a prebiotic and a beta-glucan enable to effectively treat the symptoms of HPV vaginal infections by vaginal administration.

Said composition in the invention is for use in the treatment of HPV (Papilloma virus) vaginal infections and symptoms associated thereto by topical vaginal administration.

The inventors have currently found that the composition of the invention comprising the medicinal mushroom Ganoderma Lucidum Reishi in the form of dry extract, thanks to the synergistic combination with the other active ingredients, in particular with the fat-soluble extract of echinacea, is particularly effective in fighting HPV genital infections when applied by vaginal administration.

In the present invention, "topical vaginal administration" is intended to mean topical administration directly in the perianal area, in the vulva, in the vagina and/or in the cervix.

In a preferred embodiment, the invention concerns a pharmaceutical composition for use in the treatment of HPV (Papilloma virus) vaginal infections and symptoms associated thereto by topical vaginal administration, said composition comprising
- 1-5% by weight of a dry extract of Ganoderma Lucidum Reishi with a polysaccharide titer of 50%,
- 1-3% by weight of one or more beta-glucans, more preferably 70% by weight of polysaccharides as an immunostimulant compound,
- 1-3% by weight of a prebiotic, preferably an arabinogalactan as a prebiotic, and
- 2-5% by weight of a fat-soluble extract of echinacea,
being the amounts expressed with respect to the total weight of the composition.

In a preferred embodiment, the composition of the invention is in the form of an ovule for topical vaginal administration

The invention therefore improves at least some of the disadvantages of the previous therapies and methods used, such as for example a poor bioavailability of the active ingredient, in the most affected area of the body (in this case: uterine cervix, vagina, vulva, urethra, perineum, and anus) certainly providing effective therapy. The formulation thus designed for topical use exploits the properties of the substances or vehicle thereof, and ingredients thereof, so that the pharmacological action takes place directly on the skin or mucosa where it is applied, and in this case enables a longer contact time of the active ingredient (Ganoderma Lucidum) with the affected area.

### DETAILED DESCRIPTION OF THE INVENTION

The present invention therefore concerns a composition comprising:
- from 1 to 5% by weight of one or more beta-glucans,
- from 1 to 5% by weight of a prebiotic compound,
- from 1 to 8% by weight of a dry extract of Ganoderma Lucidum Reishi, wherein said dry extract of Ganoderma Lucidum Reishi has a polysaccharide titer of 50%, and
- from 2 to 8% by weight of a fat-soluble extract of echinacea,
being the amounts expressed with respect to the total weight of the composition, and wherein said composition is for use in the treatment of HPV (Papilloma virus) vaginal infections and symptoms associated thereto by topical vaginal administration.

The composition of the invention comprises a dry extract of Ganoderma Lucidum Reishi having a polysaccharide titer of 50% and a fat-soluble extract of echinacea, in definite amounts. These extracts together with a prebiotic and a beta-glucan as immunostimulant enable to treat the symptoms of HPV infections by topical vaginal administration.

Ganoderma Lucidum Reishi is a saprophytic mushroom, or parasite, that grows and develops on buried stumps and branches or at the base of broad-leaved trunks, especially on oak and chestnut, but also poplar, ash tree, plane tree, maple, linden, beech, and others. It is an uncommon mushroom, known mostly in the East for its countless medicinal properties, but which grows, in the season from spring to autumn, even in most Italian regions. The fruiting body of Ganoderma Lucidum Reishi is characterized by a color that varies from red to dark brown (almost black) and by a "shiny" appearance, hence its scientific name: "gan" stands for shiny, "derma" means skin and "lucidum" stands for glossy. The woody consistency and the decidedly bitter taste make it an inedible mushroom.

According to the invention, the extract of Ganoderma Lucidum Reishi mushroom is an extract with a polysaccharide titer of 50% by extraction of the mushroom fruiting body. The extraction solvent is preferably a mixture of water and ethanol. Said dry extract with 50% of polysaccharides comprises a maltodextrin as excipient.

The invention provides a pharmaceutical formulation for use in the treatment of HPV vaginal infections and symptoms associated thereto. This formulation thanks to the presence of the medicinal mushroom Ganoderma Lucidum Reishi in it, by synergistic action with the other ingredients, in particular with the fat-soluble extract of echinacea in conjunction with beta-glucan as an immunostimulant and a prebiotic, is able to effectively reduce the disorders caused by the same disease, including the onset of genital warts or papilloma acuminata.

The composition of the invention comprises from 1 to 5% of one or more beta-glucans. Beta-glucans are particular immunostimulants; they can be beta glucans from different sources such as, for example, mushrooms, yeasts, cereals, algae etc. Preferably according to the invention, one or more beta-glucans comprise 70% by weight of polysaccharides.

Said composition in the invention is for use in the treatment of vaginal HPV (Papilloma virus) infections and symptoms associated thereto by topical vaginal administration.

In the present invention, "topical vaginal administration" is intended to mean the topical administration directly in the perianal area, in the vulva, in the vagina and/or in the cervix.

According to the invention, the pharmaceutical composition for use in the treatment of HPV (Papilloma virus) vaginal infections and symptoms associated thereto by topical vaginal administration preferably comprises:
- 1-5% by weight of a dry extract of Ganoderma Lucidum Reishi with a polysaccharide titer of 50%,
- 1-3% by weight of one or more beta-glucans, more preferably 70% by weight of polysaccharides as an immunostimulant compound,
- 1-3% by weight of a prebiotic, preferably an arabinogalactan as a prebiotic, and
- 2-5% by weight of a fat-soluble extract of echinacea,
being the amounts expressed with respect to the total weight of the composition. The composition for use may also include additives useful for the final formulation thereof. The composition to be administered may be prepared with any component/excipient suitable for the final formulation with the active ingredients present.

Among the excipients, it is possible to preferably mention agents capable of conferring mass, preferably semisynthetic glycerides and triglycerides; lubricating and anti-caking substances, preferably magnesium stearate, silicon dioxide; stabilizers, gelling agents and thickening agents, preferably microcrystalline cellulose; co-adjuvants in regenerative processes, moisturizing agents, preferably sodium hyaluronate; pH correctors, preferably lactic acid; soothing substances, preferably sunflower oil, avena sativa seed extract, tocopherol; and astringent agents, antibacterial agents, preferably thyme essential oil.

The composition of the invention is for use in the treatment of vaginal HPV (Papilloma virus) infections and symptoms associated thereto by topical vaginal administration.

The composition can therefore be formulated for topical vaginal administration in the form of solutions, tablets, creams, gels, suppositories, rings, and ovules.

In a preferred embodiment, the composition of the invention is in the form of ovules for topical vaginal administration.

The inventors have currently found that the composition of the invention comprising: - from 1 to 5% by weight of one or more beta-glucans, - from 1 to 5% by weight of a prebiotic compound, - from 1 to 8% by weight of a dry extract of Ganoderma Lucidum Reishi, wherein said dry extract of Ganoderma Lucidum Reishi has a polysaccharide titer of 50%, and - from 2 to 8% by weight of a fat-soluble extract of echinacea, being the amounts expressed with respect to the total weight of the composition , is particularly effective in fighting genital HPV infections when applied directly to the perianal area, vulva, vagina and/or cervix. Therefore, an aspect of the invention provides a topical composition to be used in the prevention and/or treatment of a genital disorder caused by HPV by genital administration. In the present invention there is the presence of a medicinal mushroom, with historical beneficial properties for the human body, used in an absolutely innovative way. The inventors of this innovation present a formulation comprising a dry extract of Ganoderma Lucidum Reishi in defined amounts and together with specific ingredients for topical application, in particular the fat-soluble extract of echinacea, directly on the site of action, thus promoting direct contact of all the pharmacologically active substances contained in it, in particular the β-glucans polysaccharides and the hetero-β-glucans. This strategy allowed to obtain very convincing results in the regression of symptoms associated with human HPV virus infections. Since the genital system is very sensitive to sexually transmitted infectious diseases, the immunity strengthening in this particular environment is highly required and has a very beneficial action. Local enhancement of genital immunity helps the body fight all types of genital infections, from vaginitis and nonspecific candidiasis to HPV infections. The present invention shows a further advantage, represented by the vaginal/cervical administration, which allows an easy application for the patient, and avoids the need to protect the active compounds from hostile environments such as the gastrointestinal tract. Therefore, the composition of the invention also allows a clear improvement in compliance.

The following are some examples of embodiments of the invention and evaluation of the synergy resulting from the composition of the invention, provided by way of non-limiting examples of the same invention.

### EXPERIMENTAL PART

### Example 1: preparation of the composition of the invention

For the preparation of the composition of the invention, and hence of the final product, the ingredients listed in Table 1 below were used for the preparation of a 2 gram ovule.

**Table 1**

| **Commercial name of the product** | **Chemical Class** | **mg (2 g ovule)** | **% W/W** | **Function** |
|---|---|---|---|---|
| DUB PP D1 | Semisynthetic Glycerides | 1374.0 | **70.1** | Bulking agent |
| SUPPOCIRE CM | C10-18 triglycerides | 400.0 | **20.0** | Bulking agent |
| COLLOIDAL SILICA | Silica | 10.0 | **0.5** | Anti-caking agent |
| 70% BETAGLUCAN | (1,3)-(1,6)-beta-D-glucan | 21.0 | **1.5** | Active ingredient and Immunostimulant |
| ARABINOGALACTAN | Polysaccharides | 30.0 | **1.5** | Active ingredient and Prebiotic |
| 50% GANODERMA LUCIDUM REISHI DRY EXTRACT | B-glucans, oligosaccharides | 15.0 | **1.5** | Active ingredient and Immunostimulant |
| ECHINACEA FAT-SOLUBLE EXTRACT | Sunflower oil, *Echinacea angustifolia* extract, tocopherol | 12.0 | **3.0** | Active ingredient and Healing agent, Re-epithelialization agent |
| THYME ESSENTIAL OIL | | 6.0 | **0.3** | Astringent, Antibacterial agent |
| SODIUM HYALURONATE (8-12MD) | | 10.0 | **0.5** | Regenerative processes co-adjuvant, Moisturizing agent |
| AVENA FAT-SOLUBLE EXTRACT | Sunflower oil, avena sativa seed extract, tocopherol | 1.25 | **1.0** | Soothing agent |
| 90% LACTIC ACID | Water, lactic acid | | **q.b.** | pH Corrector |
| | | **Tot:** | **100.0** | |

The ingredients were processed to produce a 2-gram ovule according to pharmaceutical formulation techniques known for the preparation of ovules.

### Example 2: evaluation of the activity of the preparation of the invention

The preparation as in example 1 was tested on a group of 15 patients with the following characteristics:
HPV positive women between the ages of 18 and 45 with a Vaginal Health Index comprised between 1 and 4. The Vaginal Health Index is a score based on 5 parameters: vaginal elasticity, fluid volume, pH, epithelial integrity and moisture on a scale of 1 to 5 (Table 2). This score allows to define the degree of atrophy in the genitourinary tract by evaluating each single parameter.

**Table 2**

| Score | 1 | 2 | 3 | 4 | 5 |
|---|---|---|---|---|---|
| Elasticity | None | Low | Fair | Good | Excellent |
| Fluid volume | None | Low amounts, fornix not fully lined | Superficial amount, fornix not fully lined | Moderate amount | Normal amount |
| pH | ≥6.1 | 5.6-6.0 | 5.1-5.5 | 4.7-50 | ≤4.6 |
| Epithelial integrity | Petechiae noticed before contact | Bleeding with light contact | Bleeding on rubbing | Fine, non-friable epithelium | Normal |
| Humidity | None, inflamed surface | None, non-inflamed surface | Minimal | Moderate | Normal |

The exclusion criteria included vaginal infections, use of vaginal products other than the investigative compound, and being pregnant or breastfeeding.

These patients took the preparation in the form of a vaginal ovule with a dosage of one ovule per day for the first 20 days and two ovules per week for the next three months.

At the end of the treatment, the results were evaluated through the evaluation of the degree of epithelialization of the cervical mucosa by standard colposcopy assigning a score from 1 (severe) to 5 (normal).

The baseline Vaginal Health Index was also assessed at the end of treatment.

### Results

A total of 15 women with a mean age of 33.1 years (range 21-44 years) completed the study following treatment with the formulation for as long as scheduled.

### Colposcopy

Treatment with the formulation as in example 1 showed a positive effect on re-epithelialization of the cervical mucosa, with a mean score of 4.61 at the final visit compared to 2.93 at baseline (P <0.001), and an overall improvement of 39%. All treated subjects showed an improvement in the epithelialization score.

### Vaginal Health Index

There was a significant increase in the Vaginal Health Index from a mean of 18.5 at baseline to 23.1 at the final visit (P = 0.005). The average vaginal pH showed a tendency to decrease, and vaginal elasticity was improved.

No adverse reactions associated to the treatment were recorded.

Based on a self-evaluation of the treated patients, the benefit obtained from the treatment was positively evaluated. Treatment compliance was found to be acceptable by the patients.

## Claims

1. A composition comprising:
- from 1 to 5% by weight of one or more beta-glucans,
- from 1 to 5% by weight of a prebiotic compound,
- from 1 to 8% by weight of a dry extract of Ganoderma Lucidum Reishi, wherein said dry extract of Ganoderma Lucidum Reishi has a polysaccharide titer of 50%, and
- from 2 to 8% by weight of a fat-soluble extract of echinacea,
being the amounts expressed with respect to the total weight of the composition, and wherein said composition is for use in the treatment of HPV (Papilloma virus) vaginal infections and symptoms associated thereto by topical vaginal administration.

2. The composition for use according to claim 1, wherein the composition comprises
- 1-5% by weight of a dry extract of Ganoderma Lucidum Reishi with a polysaccharide titer of 50%,
- 1-3% by weight of one or more beta-glucans, more preferably 70% by weight of polysaccharides as an immunostimulant compound,
- 1-3% by weight of a prebiotic, preferably an arabinogalactan as a prebiotic, and
- 2-5% by weight of a fat-soluble extract of echinacea,
being the amounts expressed with respect to the total weight of the composition.

3. The composition for use according to claim 1 or 2, wherein said composition further comprises additives/excipients selected from the group consisting of agents capable of conferring mass, preferably semisynthetic glycerides and triglycerides; lubricating and anti-caking substances, preferably magnesium stearate, silicon dioxide; stabilizers, gelling agents and thickening agents, preferably microcrystalline cellulose; co-adjuvants in regenerative processes, moisturizing agents, preferably sodium hyaluronate; pH correctors, preferably lactic acid; soothing substances, preferably sunflower oil, avena sativa seed extract, tocopherol; and astringent agents, antibacterial agents, preferably thyme essential oil.

4. The composition for use according to any one of claims 1 to 3, wherein said composition is in the pharmaceutical form of a solution, tablet, cream, gel, suppository, ring, or ovule.

5. The composition for the use of claim 4, wherein said composition is in the pharmaceutical form of an ovule.
